# EUROPEAN PATENT APPLICATION

(11) **EP 4 285 964 A1**
(43) Date of publication of application: **06.12.2023**
(21) Application number: 22305806.6
(22) Date of filing: 02.06.2022
(51) Int. Cl.: A61M 5/315

(54) **INDICATOR FOR DRUG DELIVERY DEVICE**

(71) Applicant: Becton, Dickinson and Company, Franklin Lakes, NJ 07417-1880 (US)
(72) Inventor: DUCAROUGE, Pierre, 38100 Grenoble (FR); MUNSCH, Stéphane, 38450 Vif (FR); ALLIONE, Virginie, 38100 Grenoble (FR); VALENTIN, Stéphane, 38470 L'ALBENC (FR)
(74) Representative: Germain Maureau

(57) **Abstract**

Provided herein is a subassembly for a drug delivery device, including a housing defining an interior, the housing including at least one opening therethrough, a plunger rod having a proximal end and a distal end, a drive member received in the housing interior and configured to displace the plunger rod distally, a plunger rod follower having a proximal end and a distal end, the distal end of the plunger rod follower configured to couple with the proximal end of the plunger rod, and an indicator having one or more resiliently-biased arms having a protrusion thereon, wherein the protrusion is configured to be received at least partially within the opening in the housing following activation of the drug delivery device.

## Description

### BACKGROUND OF THE INVENTION

### Field of the Invention

The present disclosure relates generally to drug delivery devices and, more specifically, to indicators for signaling the end of drug delivery.

### Description of Related Art

Drug delivery device often include indicators, whether audible, tactile, or visual, to signal end of drug delivery to users thereof. However, visual indicators, even if they're made of bright colors, are often behind a window of the device housing, making the bright color difficult for the user to see if the device is not held properly.

Accordingly, there is a need in the art for drug delivery devices with visual indicators that are highly visible, so users can properly use such devices.

### SUMMARY OF THE INVENTION

Provided herein is a subassembly for a drug delivery device, including a housing having a proximal end, a distal end, and a sidewall defining an interior, the sidewall including at least one opening therethrough, a plunger rod received at least partially in the housing interior, the plunger rod having a proximal end and a distal end, a drive member received at least partially in the housing interior and configured to displace the plunger rod distally, a plunger rod follower received at least partially in the housing interior and having a proximal end and a distal end, the distal end of the plunger rod follower configured to couple with the proximal end of the plunger rod, and an indicator received at least partially in the housing interior, including a proximal end, a distal end, and a sidewall therebetween defining an indicator interior, the indicator having one or more resiliently-biased arms having a protrusion thereon, wherein the protrusion is configured to be received at least partially within the opening in the housing sidewall following activation of the drug delivery device.

Also provided herein is a drug delivery device, including a subassembly as described herein, a lower housing, a syringe received at least partially within the lower housing, the syringe having a proximal end, a distal end, a sidewall defining an interior configured to receive a medicament, a needle arranged at the distal end of the syringe and in fluid communication with the syringe interior, and a stopper arranged at the proximal end of the syringe and slidable within the syringe interior, the stopper configured to be contacted by the distal end of the plunger rod, and a displaceable needle cover having a proximal end, a distal end comprising an opening, and a sidewall therebetween, the needle cover displaceable between a pre-use position in which the needle cover surrounds the needle, a use position in which the needle protrudes through the opening in the distal end of the needle cover, and a post-use position, in which the needle cover surrounds the needle.

### BRIEF DESCRIPTION OF THE DRAWINGS

FIG. 1 is a perspective view of a drug delivery device according to non-limiting embodiments described herein;
FIG. 2 is a cross-sectional view of a drug delivery device according to non-limiting embodiments described herein;
FIG. 3 is an exploded view of a drug delivery device according to non-limiting embodiments described herein;
FIGS. 4A and 4B are a perspective and top view of a non-limiting embodiment of an indicator member useful with a drug delivery device according to non-limiting embodiments described herein;
FIGS. 5A-5C are side cross-sectional views of a drug delivery device according to non-limiting embodiments described herein; and
FIGS. 6A-6C are perspective cross-sectional views of a drug delivery device according to non-limiting embodiments described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

It should be understood that any numerical range recited herein is intended to include all values and sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10.

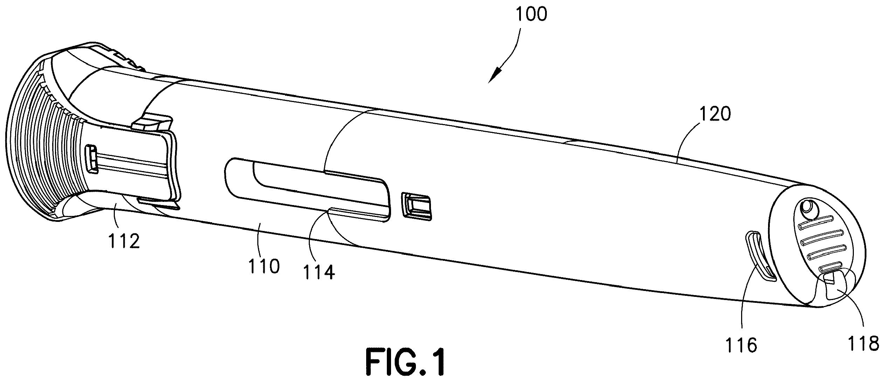

Turning to FIG. 1, shown is a drug delivery device 100, having a lower housing 110 with a cap 112 removably coupleable thereto. Lower housing 110 can at least partially define an opening, or window 114, through a sidewall thereof to allow visualization of a syringe (not shown) held in the interior of drug delivery device 100. Drug delivery device 100 may also include an upper housing 120 that at least partially defines an opening, or window 114, through a sidewall thereof, to allow visualization of a syringe (not shown) held in the interior of drug delivery device 100. Upper housing 120 may further include one or more additional windows 116, arranged in sidewall thereof at a proximal end of upper housing 120, and/or an additional window 118 arranged on proximal end of upper housing 120, optionally on a thumb pad arranged on proximal end of upper housing 120. Upper and lower housing 120, 110 may be formed of any suitable material(s) known to those of skill in the art, such as plastic(s). Windows 114, 116, 118 may be open to interior of drug delivery device 100 or may include one or more clear separators, again formed of suitable material(s) known to those of skill in the art.

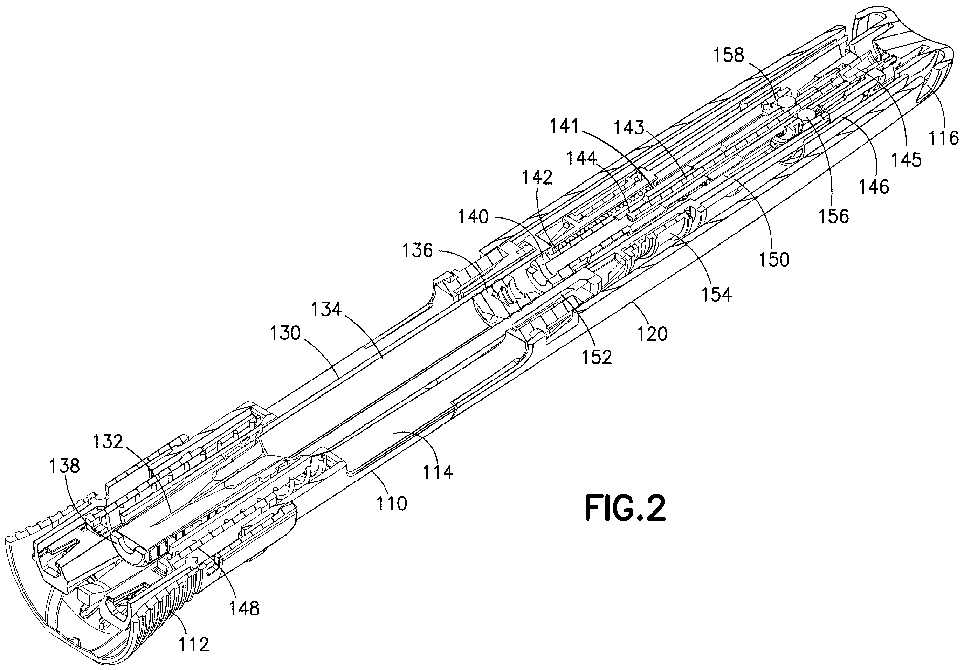

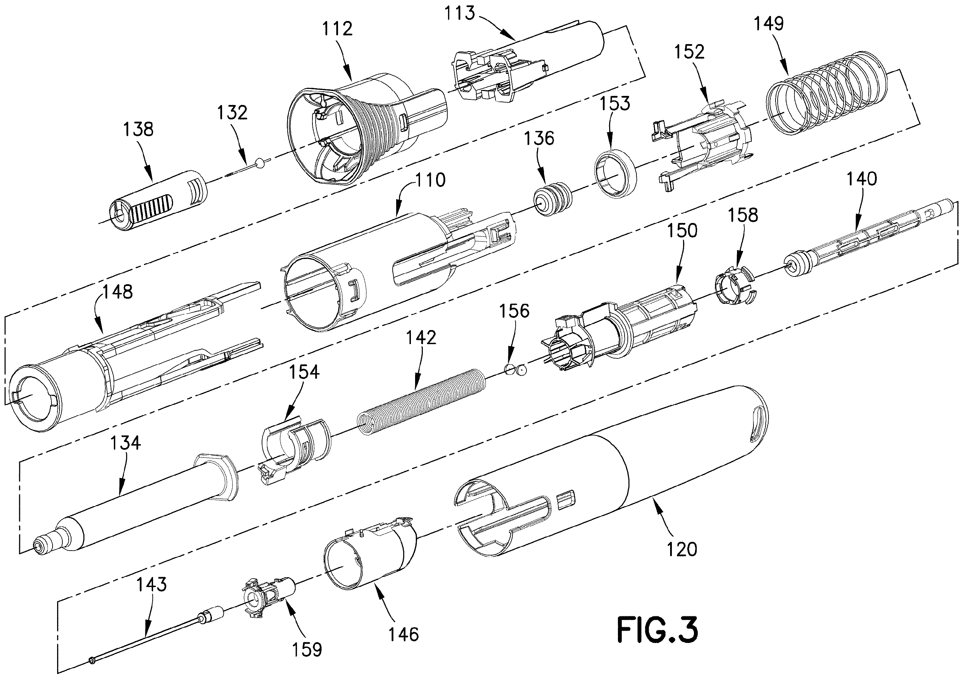

Turning to FIGS. 2 and 3, shown are a cross-sectional (FIG. 2) and exploded (FIG. 3) view of drug delivery device 100, with internal components of the device 100 visible. Drug delivery device 100 may include a syringe 130, having a proximal end having a stopper 136, a distal end having a needle 132 affixed thereto, and a barrel 134 defining an interior configured to hold one or more medicaments therein. Stopper 136 may be slidably received within syringe interior, to allow for the one or more medicaments to be expelled through needle 132. Stopper 136 may be displaced by plunger rod 140, under the influence of drive member 142. Plunger rod 140 has a proximal end, a distal end configured to engage stopper 136, and a sidewall therebetween. Plunger rod 140 may be received within (e.g., may be at least partially surrounded by) drive member 142. Suitable drive member(s) are known in the art, and can include, for example, compression springs. In non-limiting embodiments, drive member 142 is a compression spring that is biased to an expanded state, but which is maintained in a compressed state prior to activation of drug delivery device 100, which will be described below. Drive member 142 may engage plunger rod 140 by interacting with one or more protrusions on plunger rod 140.

Drive member 142 may be, prior to activation of drug delivery device 100, maintained in a compressed state, by virtue of its engagement with plunger rod 140, which is held in a proximal position by one or more ball bearings 156. Ball bearings 156 are displaceable radially outward from an inward position in which they are engaged with plunger rod 140, to an outward position in which they are no longer engaged with plunger rod 140. In this radial outward position of ball bearings 156, plunger rod 140 is released and may be displaced distally by the force of drive member 142.

Ball bearings 156 may be held in engagement with plunger rod 140 by a ring 158. Ring 158 is displaceable from a distal position, in which ring 158 is arranged between ball bearings 156 and upper housing 120, to a proximal position by engagement with displaceable container holder 150. As container holder 150 is displaced proximally during activation of drug delivery device 100, ring 158 is similarly displaced proximally, and ball bearings 156, which are biased to the outward position, move radially outwardly out of engagement with plunger rod 140. Ring 158 may include, at distal end thereof, one or more ramps, such that ball bearing(s) 156 are gradually displaced outward as ring 158 is displaced proximally. Such a design may be desirable to prevent ball bearing(s) 156 from contacting container holder 150 with sufficient force to cause an audible click which could confuse a user, who could interpret the click as an end-of-dose indicator.

Container holder 150 may comprise a proximal end, a distal end, and a sidewall therebetween defining an interior in which plunger rod 140, plunger rod follower 143, and drive member 142 may be moveably received. As noted above, container holder 150 may, at proximal end thereof, be configured to engage, and displace (as container holder 150 is displaced proximally) ring 158. At distal end thereof, container holder may be coupled to locker 154. Locker 154 and container holder 150 may be coupled in a manner such that locker 154 is rotatable relative to container holder 150. In non-limiting embodiments, locker 154 is, in a pre-activation position, engaged with one or more portions of upper housing 120. Upon being contacted by needle cover 148 during activation of drug delivery device 100 (described below), locker 154 may rotate, and thus be disengaged from upper housing 120. In non-limiting embodiments, locker 154 comprises one or more angled surfaces at distal end thereof, and one or more arms of needle cover 148, as needle cover 148 is displaced proximally, contacts the one or more ramps, causing locker 154 to rotate. Disengagement of locker 154 from upper housing 120 allows for needle cover 148, as it is displaced proximally, to displace container holder 150 (by virtue of contact between needle cover 148 and locker 154, which is coupled to container holder 150).

Needle cover 148 may include a tubular distal portion, configured to enclose needle 132, and one or more arms extending proximally from the tubular portion. Needle cover 148 may be displaceable, in a telescoping manner, relative to lower housing 110 and upper housing 120, from a pre-use position, in which needle cover 148 extends a first distance beyond a distal end of lower housing 110 and encloses needle 132 of syringe 130, to a use position, in which needle cover 148 is displaced proximally, exposing needle 132, to a post-use position, in which needle cover 148 extends a second distance beyond distal end of lower housing 110 and encloses needle 132. Needle cover 148 may be biased to a distal position by needle cover spring 149. In non-limiting embodiments, second distance is greater than first distance. In the post-use position, needle cover 148 is locked, and needle 132 cannot be re-exposed. In non-limiting embodiments, needle cover 148 is locked by engagement with one or more protrusions of engine 152. Engine 152 may include a ring at a proximal end thereof, through which syringe barrel 134 is received. In non-limiting embodiments, flanges of syringe 130 abut engine 152. In non-limiting embodiments, a ring 153 of resilient material, such a thermoplastic elastomer, is provided between engine 152 and flanges of syringe 130, to dampen forces applied to syringe 130 by virtue of plunger rod 140 displacing stopper 136 through syringe barrel 134. In non-limiting embodiments, engine 152 is engaged at one or more locations with lower housing 110, and thus is not displaceable proximally and/or distally relative to lower housing 110.

Drug delivery device 100, as noted above, may also include cap 112, which may be releasably coupleable to distal end of lower housing 110. Cap 112 may be coupled, optionally displaceably, relative to a retainer 113. Retainer 113 may, through one or more arms, engage with a rigid needle shield (RNS) 138. RNSs are known in the art, and include an inner, elastomeric portion that encases needle 132, and an outer, rigid plastic portion. In non-limiting embodiments, retainer 113 grips RNS 138 surrounding needle 132. When cap 112 is pulled distally away from lower housing 110 as drug delivery device 100 is prepared for use, retainer 113 grips the RNS 138 and removes the RNS 138, rendering the drug delivery device 100 ready for use. In non-limiting embodiments, cap 112, when in place on distal end of lower housing 110, prevents proximal displacement of needle cover 148.

With continuing reference to FIGS. 2 and 3, plunger rod may be coupled, at proximal end 141 thereof, to a plunger rod follower 143. Plunger rod follower may include a proximal end 145, a distal end 144, and may define a longitudinal axis that is coaxial with a longitudinal axis of drug delivery device 100. Distal end 144 of plunger rod follower may be configured to couple with proximal end 141 of plunger rod 140, such that as plunger rod 140 is displaced distally under the influence of drive member 142, plunger rod follower 143 is also displaced. In non-limiting embodiments, plunger rod follower 143 is received at least partially within, and telescopically with, plunger rod 140, such that during an initial stage of plunger rod 140 displacement, plunger rod follower 143 is not displaced. After a predetermined amount of displacement, distal end 144 of plunger rod follower 143 may engage proximal end 141 of plunger rod, such that plunger rod follower 143 is displaced distally, in a delayed manner relative to plunger rod 140.

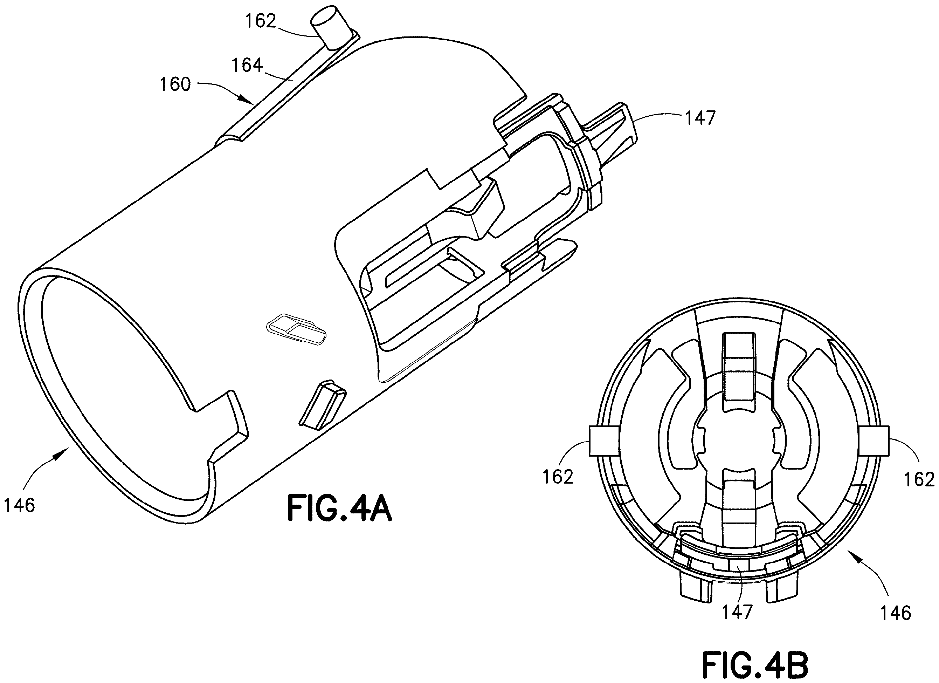

Plunger rod follower 143, at proximal end 145 thereof, may be engaged with an indicator member 146. Non-limiting embodiments of indicator member 146 are shown in FIGS. 4A and 4B. Indicator member 146 may include a proximal end, a distal end, and a sidewall therebetween defining an interior. Indicator member 146 may further include washer 159. Proximal end 145 of plunger rod follower 143 may be received within interior of indicator member 146, for example washer 159. In non-limiting embodiments, interior of indicator member 146 and/or washer 159 includes a narrowed portion at or near proximal end of indicator member 146 and/or washer 159, into which proximal end 144 of plunger rod follower 143 may be received, optionally by a friction fit.

In non-limiting embodiments, indicator assembly, including indicator member 146 and optionally washer 159, may assume a plurality of configurations. With regard to the below description, while interactions between washer 159, plunger rod follower 143, drive member 142, and upper housing 120 are exemplified, those of skill will appreciate that in non-limiting embodiments, plunger rod follower 143 may interact with indicator member 146 of indicator assembly, and indicator member 146 may interact with drive member 143 and upper housing 120, in a similar manner. In non-limiting embodiments, in a first configuration, washer 159 may have a first circumference, and in a second configuration, washer 159 may have a second circumference smaller than the first circumference. Washer 159 may be biased towards the second configuration. When proximal end 145 of plunger rod follower 143 is received within narrowed portion of washer 159, indicator member may be in first configuration (first, larger circumference). As drug delivery device 100 is activated and plunger rod 140 and plunger rod follower 143 are displaced distally by drive member 142, and proximal end 145 of plunger rod follower 143 is pulled out of engagement with narrowed portion of washer 159, washer 159 may transition to second configuration (second, smaller circumference). Washer 159, as well as upper housing 120, may be configured such that upon transitioning to second configuration, washer 159 and indicator member 146 are displaced proximally. For example, an outer perimeter of washer 159, and inner surface of upper housing 120, may have cooperating projections, which may, when washer 159 is in a first configuration, hold washer 159 against a force of drive member 142. As washer 159 transitions to second, smaller configuration, cooperation between a projection on outer surface of washer 159 and inner surface of housing 120 may cease, such that drive member 142 may, as it expands, displace washer 159 and indicator 146 proximally. In non-limiting embodiments, indicator member 146 includes an extension portion 147 that extends proximally, and, when indicator member 146 and washer 159 are displaced proximally, extension portion may become visible, optionally by at least partially entering window 118 of upper housing 120. In non-limiting embodiments, extension portion 147 protrudes at least partially through window 118, contacting a finger of a user, thereby providing a tactile indication that drug delivery is complete.

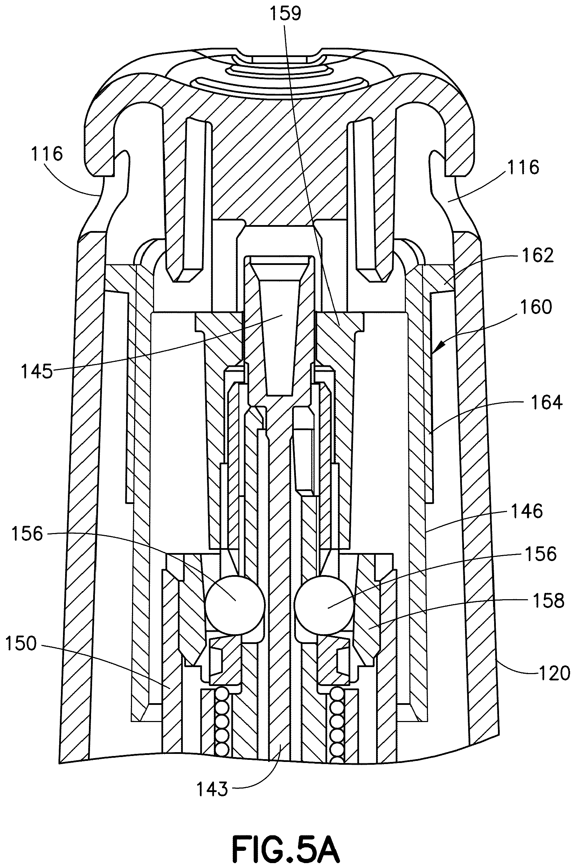

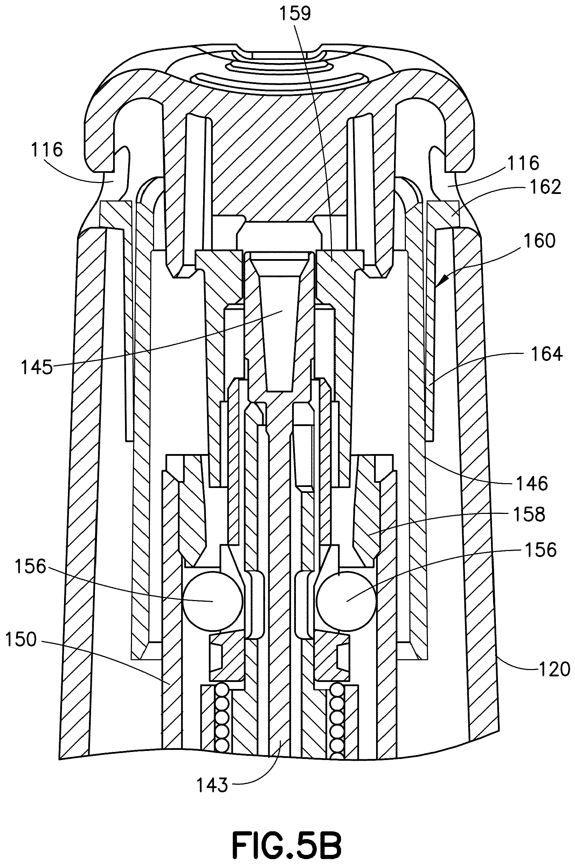

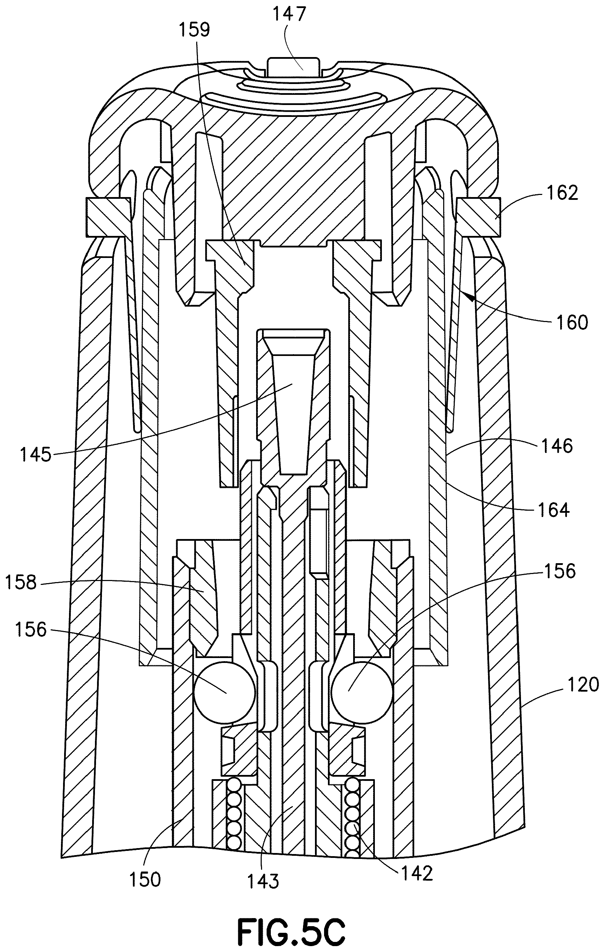

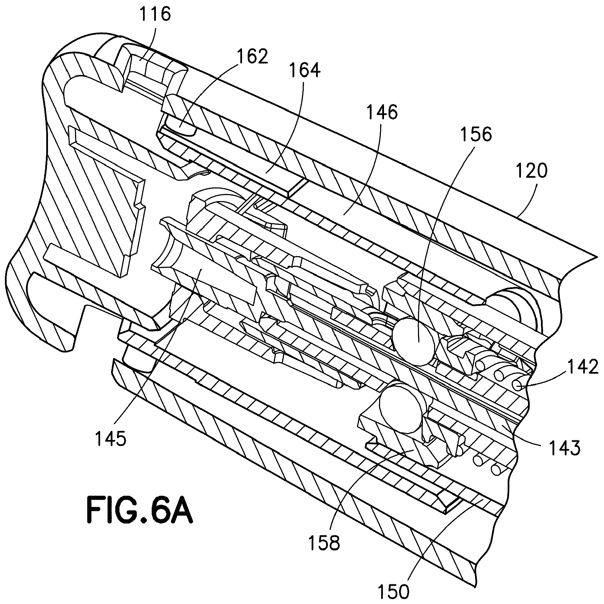

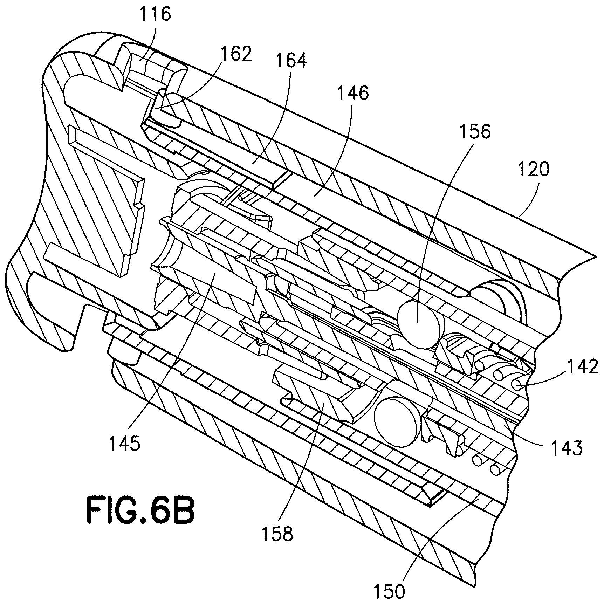

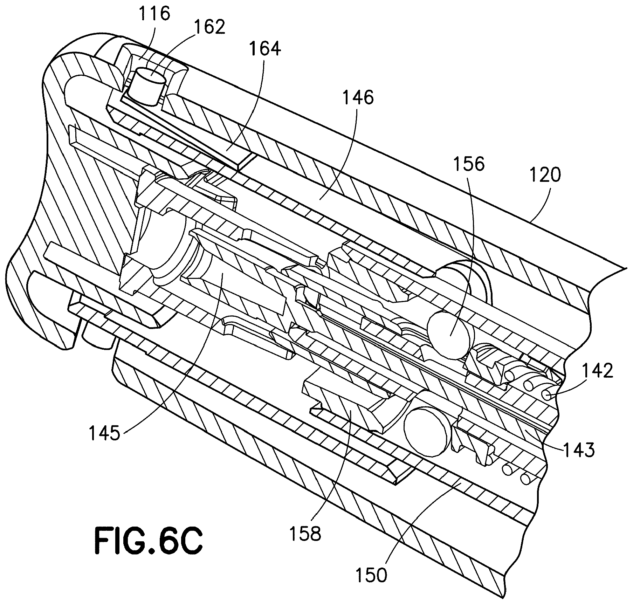

With reference to FIGS. 4-6C, shown is indicator member 146 and deployment of indicator member 146 during drug delivery. In non-limiting embodiments, indicator member 146 includes one or more outwardly-biased extension(s) 160. Extension(s) 160 may include outwardly-biased arm(s) 164, with protrusions 162 at an end thereof. In non-limiting embodiments, as shown in FIGS. 5A-6C, biased arms 164 and protrusions 162, in a pre-use state of drug delivery device 100, may be received wholly within upper housing 120. As drug delivery device 100 is activated (described below), and indicator member 146 is displaced proximally, protrusions 162 may encounter windows(s) 116 in upper housing 120, allowing biased arms 164 to flex outward, such that protrusions 162 are received at least partially within window(s) 116, optionally protruding through window(s) 116. Protrusions 162 and/or arms 164 may be of a suitable color, such that a user of drug delivery device 100 can easily view protrusions 162 and/or arms 164, and know that drug delivery has been completed. In non-limiting embodiments, protrusion(s) 162 substantially, optionally completely, fill window(s) 116.

The above-described components of drug delivery device 100 may be divided into subassemblies. For example a lower subassembly may include lower housing 110, cap 112, retainer 113, needle cover 148, needle cover spring 149, engine 152, and/or ring 153. An upper subassembly may include upper housing 120, plunger rod 140, plunger rod follower 143, container holder 150, locker 154, ring 158, washer 159, and/or indicator member 146. A syringe 130, for example a pre-filled syringe, with an RNS 138 attached thereto, may be introduced into lower subassembly, then upper subassembly may be placed onto lower subassembly for final assembly of drug delivery device 100.

With reference to the figures, activation of drug delivery device 100 will now be described. A user may grasp cap 112, and, by applying a distally-directed force, pull cap 112 distally away from lower housing 110, exposing distal end of needle cover 148. By removing cap 112, RNS 138, if present, may also be removed, through interaction between retainer 113 and RNS 138. Drug delivery device 100 may then be placed on the user's skin at a site of injection. By applying a distally-directed force to drug delivery device 100, needle cover 148 is shifted proximally, into lower housing 110, exposing needle 132 such that as needle cover 148 is further displaced proximally, needle 132 pierces the user's skin at the site of injection.

As needle cover 148 is displaced proximally, against the biasing force of needle cover spring 149, one or more arms of needle cover 148 contacts locker 154, causing locker 154 to rotate relative to upper housing 120. By virtue of this rotation, locker 154 becomes disengaged from upper housing 120, and locker 154, and container holder 150 to which locker 154 is coupled, are displaced proximally by continued proximal displacement of needle cover 148. As container holder 150 is displaced proximally, ring 158 is displaced proximally, allowing ball bearings 156 to move radially outward, freeing plunger rod 140. As plunger rod 140 is freed, drive member 142 causes distal displacement of plunger rod 140. Plunger rod 140, through its engagement at distal end thereof to stopper 136, moves stopper 136 through syringe barrel 134, causing any medicament(s) in syringe barrel 134 to be dispensed through needle 132.

As plunger rod 140 is displaced distally by drive member 142, plunger rod follower 143, which may be received within plunger rod 140, becomes engaged at its distal end 144 to plunger rod 140 after a predetermined amount of distal displacement of plunger rod 140, thereby causing distal displacement of plunger rod follower 143. As plunger rod follower 143 is displaced distally, proximal end 145 thereof, which may be engaged in a narrowed portion of indicator member 146, e.g., washer 159, is pulled out of engagement with narrowed portion of indicator member 146, e.g., washer 159. Following disengagement of proximal end 145 of plunger rod follower 143 from narrowed portion of indicator member 146, e.g., washer 159, indicator member 146, e.g., washer 159, may transition from a first configuration, in which indicator member 146 and/or washer 159 may have a first, larger circumference, to a second configuration, in which indicator member 146 and/or washer 159 may have a second, smaller circumference. Upon transitioning to second configuration, indicator member 146 and/or washer 159 are displaced proximally, and extension portion 147 may become visible, optionally by at least partially entering window 118, of upper housing 120. In addition, as indicator member 146 is displaced proximally, protrusions 162 may encounter windows(s) 116 in upper housing 120, allowing biased arms 164 of indicator member 146 to flex outwardly, such that protrusions 162 are received at least partially within window(s) 116, optionally protruding through window(s) 116.

When drug delivery is finished, drug delivery device 100 is lifted from the site of injection, needle cover 148, biased distally by needle cover spring 149, is displaced distally to a post-use position, in which needle 132 is surrounded by needle cover 148, and is locked in place through interaction with engine 152.

Although the invention has been described in detail for the purpose of illustration based on what is currently considered to be the most practical and preferred embodiments, it is to be understood that such detail is solely for that purpose and that the invention is not limited to the disclosed embodiments, but on the contrary, is intended to cover modifications and equivalent arrangements that are within the spirit and scope of the appended claims. For example, it is to be understood that the present invention contemplates that, to the extent possible, one or more features of any embodiment can be combined with one or more features of any other embodiment.

## Claims

1. A subassembly for a drug delivery device, comprising:
a housing having a proximal end, a distal end, and a sidewall defining an interior, the sidewall including at least one opening therethrough;
a plunger rod received at least partially in the housing interior, the plunger rod having a proximal end and a distal end;
a drive member received at least partially in the housing interior and configured to displace the plunger rod distally;
a plunger rod follower received at least partially in the housing interior and having a proximal end and a distal end, the distal end of the plunger rod follower configured to couple with the proximal end of the plunger rod; and
an indicator received at least partially in the housing interior, comprising a proximal end, a distal end, and a sidewall therebetween defining an indicator interior, the indicator comprising one or more resiliently-biased arms having a protrusion thereon, wherein the protrusion is configured to be received at least partially within the opening in the housing sidewall following activation of the drug delivery device.

2. The subassembly of claim 1, further comprising one or more ball bearings, the ball bearings being biased radially outward and configured to maintain the drive member in a biased configuration.

3. The subassembly of claim 2, further comprising a ring, the ring being slidable relative to the housing between a distal position, in which the ring maintains the ball bearings in a biased position, and a proximal position, in which the ball bearings are displaced radially outward.

4. The subassembly of claim 3, further comprising a container holder, the container holder configured to displace the ring from the distal position to the proximal position.

5. The subassembly of claim 1, wherein the drive member is a compression spring, and wherein, in a biased configuration, the compression spring is compressed.

6. The subassembly of claim 1, wherein the one or more resiliently-biased arms are biased radially outwardly.

7. The subassembly of claim 1, wherein the indicator is slidable relative to the housing from a distal position in which the protrusion is held within the housing interior and a proximal position in which the protrusion is received at least partially within the opening in the housing.

8. The subassembly of claim 7, wherein the proximal end of the plunger rod follower is configured to be received within a washer received within the indicator interior, the washer comprising a proximal end and a distal end.

9. The subassembly of claim 8, wherein the washer comprises, at the proximal end thereof, a narrowed portion configured to releasably receive the proximal end of the plunger rod follower.

10. The subassembly of claim 9, wherein the proximal end of the washer is biased radially inward, and wherein the proximal end of the plunger rod follower, when received within the narrowed portion, displaces the proximal end of the washer radially outward against the inward bias.

11. The subassembly of claim 8, wherein, the proximal end of the washer is configured to be displaced radially inward when the plunger rod follower is released from the narrowed portion of the proximal end of the washer.

12. The subassembly of claim 11, wherein the indicator is configured to be displaced proximally when the proximal end of the washer is displaced radially inward.

13. A drug delivery device, comprising:
the subassembly of any of claims 1-12;
a lower housing;
a syringe received at least partially within the lower housing, the syringe comprising a proximal end, a distal end, a sidewall defining an interior configured to receive a medicament, a needle arranged at the distal end of the syringe and in fluid communication with the syringe interior, and a stopper arranged at the proximal end of the syringe and slidable within the syringe interior, the stopper configured to be contacted by the distal end of the plunger rod; and
a displaceable needle cover having a proximal end, a distal end comprising an opening, and a sidewall therebetween, the needle cover displaceable between a pre-use position in which the needle cover surrounds the needle, a use position in which the needle protrudes through the opening in the distal end of the needle cover, and a post-use position, in which the needle cover surrounds the needle.

14. The drug delivery device of claim 13, wherein the needle cover is configured such that:
displacement of the needle cover from the pre-use to the use position causes displacement of the container holder, displacement of the ring, and displacement of the ball bearings, thereby causing the compression spring to expand from the biased configuration and displace the plunger rod distally.

15. The drug delivery device of claim 14, wherein, distal displacement of the plunger rod by the compression spring causes distal displacement of the plunger rod follower, causing the indicator to be displaced proximally, such that the protrusion is received at least partially within the opening in the housing.
